# EUROPEAN PATENT APPLICATION

(11) **EP 2 711 351 A1**
(43) Date of publication of application: **26.03.2014**
(21) Application number: 12185802.1
(22) Date of filing: 25.09.2012
(51) Int. Cl.: C07C 41/18, C07C 43/188, C07C 67/333, C07C 69/74, C07D 313/08, C07B 37/08, C07B 37/10, C07C 6/02, C07C 6/04, C07D 207/48, C07D 223/04, C07D 225/02, C07D 245/02, C07D 313/00, C07D 313/20, C07D 321/00, C07D 498/02

(54) **Low catalyst loading in metathesis reactions**

(71) Applicant: Evonik Industries AG, 45128 Essen (DE)
(72) Inventor: Kadyrov, Renat, 60389 Frankfurt (DE)

(57) **Abstract**

The present invention relates to a method for producing metathesis products comprising contacting metathesis starting materials under metathesis conditions with a metathesis catalyst, wherein the metathesis catalyst is employed in an amount of from 0.0001 mol-% to 1 mol-% based on half of the sum of the reactive double bonds of the metathesis starting materials and wherein the ethylene or propylene generated in the course of the reaction is removed from the reaction mixture.

## Description

The field of the invention belongs to the formation of olefins via metathesis reactions.
The present invention relates to a method for producing metathesis products comprising contacting metathesis starting materials under metathesis conditions with a metathesis catalyst, wherein the metathesis catalyst is employed in an amount lower than usual and wherein the ethylene or propylene generated in the course of the reaction is removed from the reaction mixture.

Metathesis reactions have emerged as a valuable tool in modern organic chemistry. During the last three decades so-called "well defined" catalysts have attracted much attention due to their high activity. Despite these advances, olefin metathesis is still plagued by catalyst deactivation, consequently requiring high catalyst loading. Therefore, major research efforts have been focused on increasing catalytic activity and catalyst lifetime through optimization of the ligand sphere around the metal center. The evaluation of efficiency of metathesis catalysts is typically performed under standard conditions. Several techniques were explored to perform RCM more efficiently including substrate encapsulation, microwave irradiation and use of a continuous stirred-tank reactor. Major improvements were achieved by tuning of the metathesis catalysts. Nevertheless, it is still common that catalyst loadings in a range of from 1 mol-% to 50 mol% are used in metathesis reactions. Reducing of the loading of commercially available catalysts is desired to promote this technology in industrial practice, especially where Ru catalysts are applied. A high catalyst loading is not only expensive in terms of costs but also a source of undesirable side reactions. At higher catalyst loading the metal hydride species formed from the decomposition of the metathesis catalysts promote undesirable isomerisation to a significant extent. This side reaction in olefin metathesis considerably alters the product distribution and decreases the yield of the desired product. Additionally, the side products resulting from unwanted isomerization are frequently difficult to remove using standard purification techniques.
Furthermore, high concentrations of ethylene or propylene, which are byproducts in olefin metathesis reactions, favor a high rate of nonproductive metathesis, providing additional opportunities for catalyst decomposition. Two groups previously reported a beneficial effect on metathesis performance by sparging the reaction mixture with an inert gas, but they also used catalyst loadings in a range of from 5 mol% to 22.5 mol-% for the production of tetrasubstituted double bonds (B. Nosse, A. Schall, W. B. Jeong, 0. Reiser, Adv. Synth. Catal. 2005, 347, 1869) and 1 mol-% to 10 mol-% for the production of cyclophosphamides respectively (S. R Sieck, M. D. McReynolds, C. E. Schroeder, P. R. Hanson, J. Organomet. Chem. 2006, 691, 5311).

It is therefore the task of the present invention to overcome the above-mentioned disadvantages and to significantly reduce the catalyst loading in metathesis reactions.

In one aspect the present invention relates to a method for producing metathesis products comprising contacting metathesis starting materials under metathesis conditions with a metathesis catalyst, wherein the metathesis catalyst is employed in an amount of from 0.0001 mol-% to 1 mol-% based on half of the sum of the metathesis starting materials and wherein the ethylene or propylene generated in the course of the reaction is removed from the reaction mixture.

According to the present invention the reaction temperature can be varied in a range of from 20°C to 150°C. An increase in temperature significantly enhances the reaction rates without loss of productivity (TON). Furthermore, a higher reaction temperature leads to a decrease in solubility of ethylene and propylene (Landolt-Börnstein, Zahlenwerte und Funktionen aus Physik, Chemie und Astronomie, Geophysik und Technik, IV. Band: Technik, 4. Teil: Wärmetechnik, Bandteil c: Gleichgewicht der Absorption von Gasen in Flüssigkeiten mit niedrigem Dampfdruck, H. Borcbers, H. Hausen, K.-H. Hellwege und K. Schäfer, bearbeitet von A. Kruis, Springer Verlag, Berlin, 1976). According to the present invention the preferred reaction temperature is in a range of from 50°C to 150°C, wherein a range of from 60°C to 110°C is more preferred and a temperature of 80°C is particularly preferred. According to the present invention the reaction is allowed to proceed for any suitable period of time. In some cases the reaction is allowed to proceed for 1 min, 5 min, 10 min, 20 min, 30 min, 60 min, 90 min, 2h, 3h, or 6h.
According to the present invention aliquots of the reaction mixture may be removed and analyzed by GC at an intermediate time to determine the progress of the reaction. The reaction is completed once the conversion reaches a plateau, if conversion is plotted versus time.
According to the present invention the solvent is not critical, any solvent suitable for metathesis reactions can be applied. In some cases the metathesis reaction may also be performed in the absence of any solvent. According to the present invention solvents can be selected from the group of diethyl ether, glycol, pentane, heptane, hexane, cyclohexane, petroleum ether, dichloromethane, dichlorethane, chloroform, carbon tetrachloride, dioxane, tetrahydrofuran, dimethyl sulfoxide, dimethylformamide, ethyl acetate, benzene, chlorobenzene, p-cresol, xylene, mesitylene, toluene or perfluorobenzene. Preferred solvents are heptane, dichloromethane, dichloroethane, benzene or toluene.
The reaction mixture may be agitated during metathesis reaction, which may be accomplished by stirring, shaking or any other method known to the person skilled in the art.

During the course of metathesis reactions according to the present invention considerable amounts of ethylene or propylene are generated. According to the present invention any method to remove this ethylene or propylene can be applied. Preferably, volatilization of ethylene or propylene is applied, which can be accomplished for example by vigorous stirring, by applying vacuum, i.e. by reducing the pressure of the gaseous phase above the reaction mixture, or by sparging techniques. Sparging the reaction mixture with an inert gas, wherein a gas stream is introduced into the reaction mixture, is particularly suitable to remove ethylene or propylene. Furthermore, it is preferred to continuously sparge the reaction mixture with an inert gas. Suitable inert gases are for example nitrogen or argon. Furthermore, vigorous stirring can positively influence the vacuum or sparging techniques to volatilize ethylene or propylene.

The method according to the present invention for producing metathesis products can be applied in all metathesis reactions known to those skilled in the art. Therefore, the formation of a broad variety of olefins can be accomplished by the method according to the present invention.
Depending on the type of metathesis reaction, e.g. ring-closing metathesis reaction (RCM), homo-metathesis or cross-metathesis reaction (CM), different metathesis starting materials are employed according to the present invention and different metathesis products are thus obtainable.

RCM is a variation of olefin metathesis reactions that allows the formation of cyclic olefins. RCM is an intramolecular olefin metathesis, yielding the cyclic olefin and a volatile alkene, mainly ethylene or propylene.
CM is the interchange reaction of alkylidene groups between two acyclic olefins resulting in the formation of olefins having internal double bonds. Statistically, the reaction can lead to three possible pairs of geometric isomers, i.e. E/Z pairs for two homocouplings and the cross-coupling - resulting in a total of 6 possible products.
Homometathesis is a variation of cross metathesis reactions, wherein only one olefin species is involved in the reaction.

According to the present invention irrespective of the type of metathesis reaction the term metathesis starting materials refers to any species having at least one reactive double bond in the form of an α-olefin or β-olefin, with the proviso that not more than one β-olefin is present in the same molecule, such as linear and branched-chain aliphatic olefins, cycloaliphatic olefins, aryl substituted olefins and the like, which may optionally be substituted. The total number of carbon atoms of the metathesis starting materials according to the present invention can be from 2 to 50, preferably from 4 to 25.
An α-olefin in the sense of the present invention is a 1-olefin or terminal olefin, wherein the double bond is located between first and second carbon atom of the acyclic olefin, e.g. in 1-butene or but-1-ene: CH₂=CH-CH₂-CH₃.
A β-olefin in the sense of the present invention is a 2-olefin or internal olefin, wherein the double bond is located between second and third carbon atom of the acyclic olefin, e.g. in 2-butene or but-2-ene: CH₃-CH=CH-CH₃.

According to the present invention the metathesis starting materials generally comprise molecules of general formulas I-IV

H₂C=CH-R¹-HC=CH₂ (I),

H₃C-CH=CH-R¹-HC=CH₂ (II),

H₂C=CHR² (III),

H₃C-CH=CH-CH₂-R² (IV),

wherein R¹ is selected from (C₁-C₂₄)-alkylidene, (C₁-C₂₄)-heteroalkylidene, (C₅-C₁₄)-arylidene, (C₅-C₁₄)-heteroarylyidene, (C₃-C₂₄)-cycloalkylidene, and (C₃-C₂₄)-heterocylcoalkylidene, which may each be substituted with (C₁-C₂₄)-alkyl, (C₁-C₂₄)-heteroalkyl, (C₅-C₁₄)-aryl, (C₅-C₁₄)-heteroaryl, (C₃-C₂₄)-cycloalkyl, (C₃-C₂₄)-heterocycloalkyl, F, Cl, Br, NO₂, OR', COOR', OCOOR', NHCOOR', CONH₂, CONHR', CONR'₂, SO₂R', NHSO₂R', P(O)(OR')₂, and NHP(O)(OR')₂, wherein R' is selected from (C₁-C₂₄)-alkyl, (C₁-C₂₄) -heteroalkyl, (C₅-C₁₄) -aryl, (C₅-C₁₄)-heteroaryl, (C₃-C₂₄)-cycloalkyl, and (C₃-C₂₄)-heterocylcoalkyl, preferably R' is selected from Me, Et, n-Pr, i-Pr, n-Bu, Bn, Ph and p-MeC₆H₅;
and R² is selected from H, (C₁-C₂₄)-alkyl, (C₁-C₂₄)-heteroalkyl, (C₅-C₁₄)-aryl, (C₅-C₁₄)-heteroaryl, (C₃-C₂₄)-cycloalkyl, and (C₃-C₂₄)-heterocylcoalkyl, which may each be substituted with C₁-C₂₄)-alkyl, (C₁-C₂₄) -heteroalkyl, (C₅-C₁₄) -aryl, (C₅-C₁₄)-heteroaryl, (C₃-C₂₄)-cycloalkyl, (C₃-C₂₄)-heterocycloalkyl, F, Cl, BR, NO₂, OR', COOR', OCOOR', NHCOOR', CONH₂, CONHR', CONR' ₂, SO₂R', NHSO₂R', P(O)(OR')₂, and NHP(O)(OR')₂, wherein R' is selected from (C₁-C₂₄)-alkyl, (C₁-C₂₄)-heteroalkyl, (C₅-C₁₄)-aryl, (C₅-C₁₄)-heteroaryl, (C₃-C₂₄)-cycloalkyl, and (C₃-C₂₄)-heterocylcoalkyl, preferably R' is selected from Me, Et, n-Pr, i-Pr, n-Bu, Bn, Ph and p-MeC₆H₅.

According to the present invention a (C₁-C₂₄)-alkyl group or (C₁-C₂₄)-alkylidene bridge is a linear or branched-chain alkyl group, which may be substituted as described before, wherein the sum of the carbon atoms is 1-24. Branched-chain alkyl groups may exhibit the branch at any carbon atom. Preferred are linear (C₁-C₂₂)-alkyl groups, e.g. methyl, ethyl, n-propyl, n-butyl, n-pentyl, n-hexyl, n-heptyl, n-octyl, nonyl, n-decyl, n-undecyl, n-dodecyl, n-tetradecyl, n-pentadecyl, n-hexydecyl, n-heptadecyl, n-octadecyl, n-nonadecyl, n-icosyl, which may each be substituted as described before. Even more preferred are linear (C₃-C₁₈)-alkyl groups, which may each be substituted as described before.
According to the present invention a (C₅-C₁₄)-aryl group or (C₅-C₁₄)-aryl bridge is a cyclic aromatic system with 5-14 carbon atoms, wherein mono-, and bi-cyclic aromatic systems are included, which may each be substituted as described before. Preferred are (C₅-C₈)-monocyclic aryl groups, e.g. phenyl, and (C₁₀-C₁₄)-bicyclic aryl groups, e.g. napththyl, which may each be substituted as described before.
According to the present invention a (C₃-C₂₄)-cycloalkyl group or (C₃-C₂₄)-cycloalkyl bridge is a cyclic alkyl group with 3-24 carbon atoms, wherein mono-, bi- and tri-cyclic alkyl groups are included, which may each be substituted as described before. Preferred are (C₃-C₁₀)-cycloalkyl groups, e.g. cyclopropyl, cyclobutyl, cyclopentyl, cyclohexyl, cycloheptyl, cyclooctyl, cyclononyl, and cyclodecyl, which may each be substituted as described before.
According to the present invention a (C₁-C₂₄)-heteroalkyl group or (C₁-C₂₄)-heteroalkyl bridge is a (C₁-C₂₄)-alkyl group as described before, wherein the sum of the atoms is 1-24 and at least one carbon atom is exchanged by a heteroatom selected from O, N, and S, and/or a functional group selected from -NH- , -NTs-, -NBoc-, -C(=O)-, -C(=O)O-, -C(=O)NH-, -C(=O)NTs-, - C(=O)NBoc-, -S(O)-, -S(=O)NH-, -P(=O)-, -P(=O)O- and -P(=O)NH-.
According to the present invention a (C₅-C₁₄)-heteroaryl group is a (C₅-C₁₄)-aryl group as described before, wherein the sum of the atoms is 5-14 and at least one carbon atom is exchanged by a heteroatom selected from O, N, and S, and/or a functional group selected from -NH-, -NTs-, -NBoc-, -C(=O)-, -C(=O)O-, - C(=O)NH-, -C(=O)NTs-, -C(=O)NBoc-, -S(O)-, -S(=O)NH-,-P(=O)-, -P(=O)O- and -P (=O) NH-. Preferred are furan, thiophene, pyrrole, pyridine, indole.
According to the present invention a (C₃-C₂₄)-heterocycloalkyl group or (C₃-C₂₄)-heterocycloalkyl bridge is a (C₃-C₂₄)-cycloalkyl group as described before, wherein the sum of the atoms is 3-24 and at least one carbon atom is exchanged by a heteroatom selected from O, N, and S, and/or a functional group selected from -NH-, -NTs-, -NBoc-, -C(=O)-, -C(=O)O-, - C(=O)NH-, -C(=O)NTs-, -C(=O)NBoc-, -S(O)-, -S(=O)NH-,-P(=O)-, -P(=O)O- and -P(=O)NH-. Preferred are (C₃-C₈)-heterocycloalkyl groups, like pyrrolidyl, piperidyl, tetrahydrofuryl.

According to the present invention preferred metathesis starting materials are those of general formulas I and II, having two reactive double bonds in the form of two α-olefins or one α-olefin and one β-olefin. Even more preferred metathesis starting materials according to the present invention are those of general formulas I and II having a linear (C₁-C₂₄)-alkylidene chain or a linear (C₁-C₂₄)-heteroalkylidene chain, wherein (C₁-C₂₄)-alkylidene and (C₁-C₂₄)-heteroalkylidene are defined as described before. Particularly preferred metathesis starting materials according to the present invention are those of general formulas I and II having a linear (C₃-C₁₈)-alkylidene chain or a linear (C₃-C₁₈)-heteroalkylidene chain with 1-5 heteroatoms, wherein (C₃-C₁₈)-alkylidene and (C₃-C₁₈)-heteroalkylidene are defined as described before, and wherein further the heteroatoms are independently selected from O and N and wherein the heteroatoms are part of a functional group selected from -NH-, -NTs-, -NBoc-, -C(=O)-, -C(=O)O-, -C(=O)NH-, -C(=O)NTs-, - C(=O)NBoc-.

According to the present invention preferred metathesis products are cyclic olefins. These cyclic olefins exhibit a 4-to 50-membered ring system, more preferably the olefins exhibit a 5- to 20-membered ring system. The cyclic olefin can be either homocyclic or heterocyclic, wherein homocyclic describes a ring system of carbon atoms, and heterocyclic describes a ring system of carbon atoms together with at least one heteroatom selected from O, N, and S and/or a functional group selected from -NH-, -NTs-, -NBoc-, -C(=O)-, -C(=O)O-, - C(=O)NH-, -C(=O)NTs-, -C(=O)NBoc-, -S(O)-, -S(=O)NH-,-P(=O)-, -P(=O)O- and -P(=O)NH-. Preferably, a heterocyclic olefin according to the present invention exhibits a 5- to 20-membered ring system with 1 to 5 heteroatoms, wherein the heteroatoms are independently selected from O and N and wherein the heteroatoms are part of a functional group selected from -NH-, -NTs-, -NBoc-, -C(=O)-, -C(=O)O-, - C(=O)NH-, -C(=O)NTs-, -C(=O)NBoc-.
Examples of metathesis starting materials which are suitable for metathesis reactions according to the present invention are olefins, like 1-olefins and 2-olefins, and dienes of 1-olefins or a combination of 1-olefin and 2-olefin, wherein dienes with terminal double bonds (1-olefins) are preferred. According to the present invention metathesis starting materials leading to cyclic olefins are acyclic olefins having two reactive double bonds in the form of two α-olefins, or one α-olefin and one β-olefin. Particularly preferred are metathesis starting materials having a chain of 4 to 25 atoms, which is optionally substituted, and having two reactive double bonds in the form of two α-olefins, or one α-olefin and one β-olefin.

According to the present invention the metathesis starting material concentration is in a range of from 0.2 mM to 400 mM, preferably from 5 mM to 40 mM, more preferably from 8 mM to 40 mM, particularly preferred is a range of from 8 mM to 20 mM. According to the present invention the stoichiometry of starting materials is the same as for other olefin metathesis reactions: for cross-metathesis reactions equimolar amounts or a slight excess of one starting material is applied.

The method of the present invention is not limited to specific metathesis catalysts. According to the present invention any catalyst suitable for metathesis reactions can be applied. Particularly suitable are catalysts which are selected from group 1, which consists of catalysts of general formulas 1, 2, 3 and so called "ill defined" metathesis catalysts, e.g. WCl₆/SnBu₄, WOCl₄/EtAlCl₂), MoO₃/SiO₂, Re₂O₇/Al₂O₃) (K. J. Ivin, Olefin Metathesis, Academic Press, London, 1983). The molybdenum and tungsten alkylidenes of the general formulas 1 and 2 are active in metathesis transformations (R. R. Schrock, Tetrahedron 1999, 55, 8141). Ruthenium catalysts of general formula 3 are particularly preferred due to their ability to tolerate polar functional groups (G. C. Vougioukalakis, R. H. Grubbs, Chem. Rev. 2010, 110, 1746).

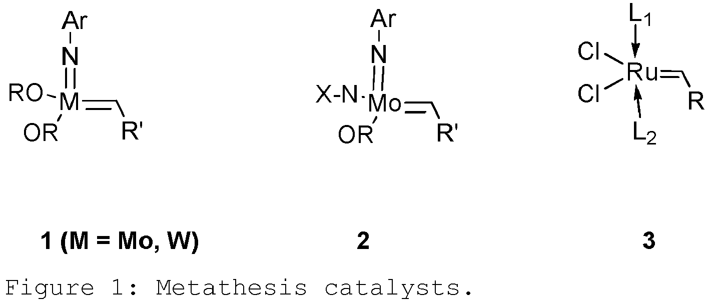

In general formulas 1-3 of figure 1,
each R,R' is selected from (C₅-C₁₄)-aryl and (C₁-C₂₄)-alkyl, each Ar is selected from (C₅-C₁₄)-aryl,
X-N is selected from (C₃-C₈)-heterocycloalkyl, like pyrrolidyl or piperidyl,
L₁, L₂ are each independently selected fom neutral electron donor ligands, like phosphines and N-heterocyclic carbenes (NHC),
wherein (C₅-C₁₄) -aryl, (C₁-C₂₄) -alkyl and (C₃-C₈)-heterocycloalkyl is defined as described before.
In general formula 1 of figure 1 R is preferably selected from t-Bu, CMe (CF₃) ₂, SiMe₃ and 2,6-diisopropylphenyl, R' is preferably selected from t-Bu, CMe₂Ph and neopentyl, and Ar is preferably selected from 2,6-dimethylphenyl and 2,6-diisopropylphenyl.
In general formula 2 of figure 1 R is preferably selected from t-Bu, CMe(CF₃)₂, SiMe₃ and 2,6-di(2',4',6'-triisopropylphenyl)phenyl, R' is preferably selected from t-Bu, CMe₂Ph and neopentyl, Ar is selected from 2,6-dimethylphenyl and 2,6-diisopropylphenyl, and X-N is preferably selected from pyrrol and 2,5-dimethylpyrrol.
In general formula 3 of figure 1 R is preferably selected from phenyl, 2,2'-dimethylvinyl and thienyl, L₁ is selected from PPh₃ and PCy₃, and L₂ is selected from PCy₃, 1,3-bis(2,4,6-trimethylphenyl)imidazol-2-ylidene, 1,3-bis(2,4,6-trimethylphenyl)-4,5-dimethylimidazol-2-ylidene, 1,3-bis(2,6-diisopropylphenyl)imidazol-2-ylidene, 1,3-bis(2,4,6-trimethylphenyl)-4,5-dihydroimidazol-2-ylidene and 1,3-bis(2,6-diisopropylphenyl)-4,5-dihydroimidazol-2-ylidene.

Remarkably results have been achieved by applying commercially available Ru-catalysts of formulas A-D (see Figure 2, experimental section), which are special embodiments of catalysts according to general formula 3.

According to the present invention irrespective of the constitution of the metathesis catalyst, the metathesis catalyst is employed in the metathesis reaction in an amount of from 1 ppm to 1 mol-% based on half of the sum of the reactive double bonds of the metathesis starting materials. The unit ppm has to be understood as follows 1 ppm = 0.0001 mol-%. According to the present invention preferably, the catalyst is employed in an amount of from 50 ppm - 2000 ppm, more preferably, the catalyst is employed in an amount of from 50 ppm to 500 ppm.

Table 1 lists reaction conditions that can be employed in embodiments of the method of the present invention.

**Table 1**

| **type of catalyst** | **amount of catalyst [ppm]** | **concentration of metathesis starting materials [mM]** | **method to remove ethylene or propylene** | **metathesis conditions: solvent = toluol; stirring 800 rpm** |
|---|---|---|---|---|
| Catalysts of Group 1 | 1-10000 | 0.2-4000 | Vacuum | 20-150°C |
| Ru catalysts of formula 3 | 1-10000 | 0.2-4000 | Vacuum | 20-150°C |
| Catalysts of formulas A,B,C,D | 1-10000 | 0.2-4000 | Vacuum | 20-150°C |
| Catalysts of Group 1 | 1-10000 | 0.2-4000 | Sparging inert gas | 20-150°C |
| Ru catalysts of formula 3 | 1-10000 | 0.2-4000 | Sparging inert gas | 20-150°C |
| Catalysts of formulas A,B,C,D | 1-10000 | 0.2-4000 | Sparging inert gas | 20-150°C |
| Catalysts of Group 1 | 1-10000 | 0.2-4000 | Sparging argon | 20-150°C |
| Ru catalysts of formula 3 | 1-10000 | 0.2-4000 | Sparging argon | 20-150°C |
| Catalysts of formulas A,B,C,D | 1-10000 | 0.2-4000 | Sparging argon | 20-150°C |
| Catalysts of Group 1 | 50-2000 | 0.2-4000 | Vacuum | 20-150°C |
| Ru catalysts of formula 3 | 50-2000 | 0.2-4000 | Vacuum | 20-150°C |
| Catalysts of formulas A,B,C,D | 50-2000 | 0.2-4000 | Vacuum | 20-150°C |
| Catalysts of Group 1 | 50-2000 | 0.2-4000 | Sparging inert gas | 20-150°C |
| Ru catalysts of formula 3 | 50-2000 | 0.2-4000 | Sparging inert gas | 20-150°C |
| Catalysts of formulas A,B,C,D | 50-2000 | 0.2-4000 | Sparging inert gas | 20-150°C |
| Catalysts of Group 1 | 50-2000 | 0.2-4000 | Sparging argon | 20-150°C |
| Ru catalysts of formula 3 | 50-2000 | 0.2-4000 | Sparging argon | 20-150°C |
| Catalysts of formulas A,B,C,D | 50-2000 | 0.2-4000 | Sparging argon | 20-150°C |
| Catalysts of Group 1 | 50-500 | 0.2-4000 | Vacuum | 20-150°C |
| Ru catalysts of formula 3 | 50-500 | 0.2-4000 | Vacuum | 20-150°C |
| Catalysts of formulas A,B,C,D | 50-500 | 0.2-4000 | Vacuum | 20-150°C |
| Catalysts of Group 1 | 50-500 | 0.2-4000 | Sparging inert gas | 20-150°C |
| Ru catalysts of formula 3 | 50-500 | 0.2-4000 | Sparging inert gas | 20-150°C |
| Catalysts of formulas A,B,C,D | 50-500 | 0.2-4000 | Sparging inert gas | 20-150°C |
| Catalysts of Group 1 | 50-500 | 0.2-4000 | Sparging argon | 20-150°C |
| Ru catalysts of formula 3 | 50-500 | 0.2-4000 | Sparging argon | 20-150°C |
| Catalysts of formulas A,B,C,D | 50-500 | 0.2-4000 | Sparging argon | 20-150°C |
| Catalysts of Group 1 | 1-10000 | 5-40 | Vacuum | 20-150°C |
| Ru catalysts of formula 3 | 1-10000 | 5-40 | Vacuum | 20-150°C |
| Catalysts of formulas A,B,C,D | 1-10000 | 5-40 | Vacuum | 20-150°C |
| Catalysts of Group 1 | 1-10000 | 5-40 | Sparging inert gas | 20-150°C |
| Ru catalysts of formula 3 | 1-10000 | 5-40 | Sparging inert gas | 20-150°C |
| Catalysts of formulas A,B,C,D | 1-10000 | 5-40 | Sparging inert gas | 20-150°C |
| Catalysts of Group 1 | 1-10000 | 5-40 | Sparging argon | 20-150°C |
| Ru catalysts of formula 3 | 1-10000 | 5-40 | Sparging argon | 20-150°C |
| Catalysts of formulas A,B,C,D | 1-10000 | 5-40 | Sparging argon | 20-150°C |
| Catalysts of Group 1 | 50-2000 | 5-40 | Vacuum | 20-150°C |
| Ru catalysts of formula 3 | 50-2000 | 5-40 | Vacuum | 20-150°C |
| Catalysts of formulas A,B,C,D | 50-2000 | 5-40 | Vacuum | 20-150°C |
| Catalysts of Group 1 | 50-2000 | 5-40 | Sparging inert gas | 20-150°C |
| Ru catalysts of formula 3 | 50-2000 | 5-40 | Sparging inert gas | 20-150°C |
| Catalysts of formulas A,B,C,D | 50-2000 | 5-40 | Sparging inert gas | 20-150°C |
| Catalysts of Group 1 | 50-2000 | 5-40 | Sparging argon | 20-150°C |
| Ru catalysts of formula 3 | 50-2000 | 5-40 | Sparging argon | 20-150°C |
| Catalysts of formulas A,B,C,D | 50-2000 | 5-40 | Sparging argon | 20-150°C |
| Catalysts of Group 1 | 50-500 | 5-40 | Vacuum | 20-150°C |
| Ru catalysts of formula 3 | 50-500 | 5-40 | Vacuum | 20-150°C |
| Catalysts of formulas A,B,C,D | 50-500 | 5-40 | Vacuum | 20-150°C |
| Catalysts of Group 1 | 50-500 | 5-40 | Sparging inert gas | 20-150°C |
| Ru catalysts of formula 3 | 50-500 | 5-40 | Sparging inert gas | 20-150°C |
| Catalysts of formulas A,B,C,D | 50-500 | 5-40 | Sparging inert gas | 20-150°C |
| Catalysts of Group 1 | 50-500 | 5-40 | Sparging argon | 20-150°C |
| Ru catalysts of formula 3 | 50-500 | 5-40 | Sparging argon | 20-150°C |
| Catalysts of formulas A,B,C,D | 50-500 | 5-40 | Sparging argon | 20-150°C |
| Catalysts of Group 1 | 1-10000 | 8-40 | Vacuum | 20-150°C |
| Ru catalysts of formula 3 | 1-10000 | 8-40 | Vacuum | 20-150°C |
| Catalysts of formulas A,B,C,D | 1-10000 | 8-40 | Vacuum | 20-150°C |
| Catalysts of Group 1 | 1-10000 | 8-40 | Sparging inert gas | 20-150°C |
| Ru catalysts of formula 3 | 1-10000 | 8-40 | Sparging inert gas | 20-150°C |
| Catalysts of formulas A,B,C,D | 1-10000 | 8-40 | Sparging inert gas | 20-150°C |
| Catalysts of Group 1 | 1-10000 | 8-40 | Sparging argon | 20-150°C |
| Ru catalysts of formula 3 | 1-10000 | 8-40 | Sparging argon | 20-150°C |
| Catalysts of formulas A,B,C,D | 1-10000 | 8-40 | Sparging argon | 20-150°C |
| Catalysts of Group 1 | 50-2000 | 8-40 | Vacuum | 20-150°C |
| Ru catalysts of formula 3 | 50-2000 | 8-40 | Vacuum | 20-150°C |
| Catalysts of formulas A,B,C,D | 50-2000 | 8-40 | Vacuum | 20-150°C |
| Catalysts of Group 1 | 50-2000 | 8-40 | Sparging inert gas | 20-150°C |
| Ru catalysts of formula 3 | 50-2000 | 8-40 | Sparging inert gas | 20-150°C |
| Catalysts of formulas A,B,C,D | 50-2000 | 8-40 | Sparging inert gas | 20-150°C |
| Catalysts of Group 1 | 50-2000 | 8-40 | Sparging argon | 20-150°C |
| Ru catalysts of formula 3 | 50-2000 | 8-40 | Sparging argon | 20-150°C |
| Catalysts of formulas A,B,C,D | 50-2000 | 8-40 | Sparging argon | 20-150°C |
| Catalysts of Group 1 | 50-500 | 8-40 | Vacuum | 20-150°C |
| Ru catalysts of formula 3 | 50-500 | 8-40 | Vacuum | 20-150°C |
| Catalysts of formulas A,B,C,D | 50-500 | 8-40 | Vacuum | 20-150°C |
| Catalysts of Group 1 | 50-500 | 8-40 | Sparging inert gas | 20-150°C |
| Ru catalysts of formula 3 | 50-500 | 8-40 | Sparging inert gas | 20-150°C |
| Catalysts of formulas A,B,C,D | 50-500 | 8-40 | Sparging inert gas | 20-150°C |
| Catalysts of Group 1 | 50-500 | 8-40 | Sparging argon | 20-150°C |
| Ru catalysts of formula 3 | 50-500 | 8-40 | Sparging argon | 20-150°C |
| Catalysts of formulas A,B,C,D | 50-500 | 8-40 | Sparging argon | 20-150°C |
| Catalysts of Group 1 | 1-10000 | 8-20 | Vacuum | 20-150°C |
| Ru catalysts of formula 3 | 1-10000 | 8-20 | Vacuum | 20-150°C |
| Catalysts of formulas A,B,C,D | 1-10000 | 8-20 | Vacuum | 20-150°C |
| Catalysts of Group 1 | 1-10000 | 8-20 | Sparging inert gas | 20-150°C |
| Ru catalysts of formula 3 | 1-10000 | 8-20 | Sparging inert gas | 20-150°C |
| Catalysts of formulas A,B,C,D | 1-10000 | 8-20 | Sparging inert gas | 20-150°C |
| Catalysts of Group 1 | 1-10000 | 8-20 | Sparging argon | 20-150°C |
| Ru catalysts of formula 3 | 1-10000 | 8-20 | Sparging argon | 20-150°C |
| Catalysts of formulas A,B,C,D | 1-10000 | 8-20 | Sparging argon | 20-150°C |
| Catalysts of Group 1 | 50-2000 | 8-20 | Vacuum | 20-150°C |
| Ru catalysts of formula 3 | 50-2000 | 8-20 | Vacuum | 20-150°C |
| Catalysts of formulas A,B,C,D | 50-2000 | 8-20 | Vacuum | 20-150°C |
| Catalysts of Group 1 | 50-2000 | 8-20 | Sparging inert gas | 20-150°C |
| Ru catalysts of formula 3 | 50-2000 | 8-20 | Sparging inert gas | 20-150°C |
| Catalysts of formulas A,B,C,D | 50-2000 | 8-20 | Sparging inert gas | 20-150°C |
| Catalysts of Group 1 | 50-2000 | 8-20 | Sparging argon | 20-150°C |
| Ru catalysts of formula 3 | 50-2000 | 8-20 | Sparging argon | 20-150°C |
| Catalysts of formulas A,B,C,D | 50-2000 | 8-20 | Sparging argon | 20-150°C |
| Catalysts of Group 1 | 50-500 | 8-20 | Vacuum | 20-150°C |
| Ru catalysts of formula 3 | 50-500 | 8-20 | Vacuum | 20-150°C |
| Catalysts of formulas A,B,C,D | 50-500 | 8-20 | Vacuum | 20-150°C |
| Catalysts of Group 1 | 50-500 | 8-20 | Sparging inert gas | 20-150°C |
| Ru catalysts of formula 3 | 50-500 | 8-20 | Sparging inert gas | 20-150°C |
| Catalysts of formulas A,B,C,D | 50-500 | 8-20 | Sparging inert gas | 20-150°C |
| Catalysts of Group 1 | 50-500 | 8-20 | Sparging argon | 20-150°C |
| Ru catalysts of formula 3 | 50-500 | 8-20 | Sparging argon | 20-150°C |
| Catalysts of formulas A,B,C,D | 50-500 | 8-20 | Sparging argon | 20-150°C |
| Catalysts of Group 1 | 1-10000 | 0.2-4000 | Vacuum | 50-150°C |
| Ru catalysts of formula 3 | 1-10000 | 0.2-4000 | Vacuum | 50-150°C |
| Catalysts of formulas A,B,C,D | 1-10000 | 0.2-4000 | Vacuum | 50-150°C |
| Catalysts of Group 1 | 1-10000 | 0.2-4000 | Sparging inert gas | 50-150°C |
| Ru catalysts of formula 3 | 1-10000 | 0.2-4000 | Sparging inert gas | 50-150°C |
| Catalysts of formulas A,B,C,D | 1-10000 | 0.2-4000 | Sparging inert gas | 50-150°C |
| Catalysts of Group 1 | 1-10000 | 0.2-4000 | Sparging argon | 50-150°C |
| Ru catalysts of formula 3 | 1-10000 | 0.2-4000 | Sparging argon | 50-150°C |
| Catalysts of formulas A,B,C,D | 1-10000 | 0.2-4000 | Sparging argon | 50-150°C |
| Catalysts of Group 1 | 50-2000 | 0.2-4000 | Vacuum | 50-150°C |
| Ru catalysts of formula 3 | 50-2000 | 0.2-4000 | Vacuum | 50-150°C |
| Catalysts of formulas A,B,C,D | 50-2000 | 0.2-4000 | Vacuum | 50-150°C |
| Catalysts of Group 1 | 50-2000 | 0.2-4000 | Sparging inert gas | 50-150°C |
| Ru catalysts of formula 3 | 50-2000 | 0.2-4000 | Sparging inert gas | 50-150°C |
| Catalysts of formulas A,B,C,D | 50-2000 | 0.2-4000 | Sparging inert gas | 50-150°C |
| Catalysts of Group 1 | 50-2000 | 0.2-4000 | Sparging argon | 50-150°C |
| Ru catalysts of formula 3 | 50-2000 | 0.2-4000 | Sparging argon | 50-150°C |
| Catalysts of formulas A,B,C,D | 50-2000 | 0.2-4000 | Sparging argon | 50-150°C |
| Catalysts of Group 1 | 50-500 | 0.2-4000 | Vacuum | 50-150°C |
| Ru catalysts of formula 3 | 50-500 | 0.2-4000 | Vacuum | 50-150°C |
| Catalysts of formulas A,B,C,D | 50-500 | 0.2-4000 | Vacuum | 50-150°C |
| Catalysts of Group 1 | 50-500 | 0.2-4000 | Sparging inert gas | 50-150°C |
| Ru catalysts of formula 3 | 50-500 | 0.2-4000 | Sparging inert gas | 50-150°C |
| Catalysts of formulas A,B,C,D | 50-500 | 0.2-4000 | Sparging inert gas | 50-150°C |
| Catalysts of Group 1 | 50-500 | 0.2-4000 | Sparging argon | 50-150°C |
| Ru catalysts of formula 3 | 50-500 | 0.2-4000 | Sparging argon | 50-150°C |
| Catalysts of formulas A,B,C,D | 50-500 | 0.2-4000 | Sparging argon | 50-150°C |
| Catalysts of Group 1 | 1-10000 | 5-40 | Vacuum | 50-150°C |
| Ru catalysts of formula 3 | 1-10000 | 5-40 | Vacuum | 50-150°C |
| Catalysts of formulas A,B,C,D | 1-10000 | 5-40 | Vacuum | 50-150°C |
| Catalysts of Group 1 | 1-10000 | 5-40 | Sparging inert gas | 50-150°C |
| Ru catalysts of formula 3 | 1-10000 | 5-40 | Sparging inert gas | 50-150°C |
| Catalysts of formulas A,B,C,D | 1-10000 | 5-40 | Sparging inert gas | 50-150°C |
| Catalysts of Group 1 | 1-10000 | 5-40 | Sparging argon | 50-150°C |
| Ru catalysts of formula 3 | 1-10000 | 5-40 | Sparging argon | 50-150°C |
| Catalysts of formulas A,B,C,D | 1-10000 | 5-40 | Sparging argon | 50-150°C |
| Catalysts of Group 1 | 50-2000 | 5-40 | Vacuum | 50-150°C |
| Ru catalysts of formula 3 | 50-2000 | 5-40 | Vacuum | 50-150°C |
| Catalysts of formulas A,B,C,D | 50-2000 | 5-40 | Vacuum | 50-150°C |
| Catalysts of Group 1 | 50-2000 | 5-40 | Sparging inert gas | 50-150°C |
| Ru catalysts of formula 3 | 50-2000 | 5-40 | Sparging inert gas | 50-150°C |
| Catalysts of formulas A,B,C,D | 50-2000 | 5-40 | Sparging inert gas | 50-150°C |
| Catalysts of Group 1 | 50-2000 | 5-40 | Sparging argon | 50-150°C |
| Ru catalysts of formula 3 | 50-2000 | 5-40 | Sparging argon | 50-150°C |
| Catalysts of formulas A,B,C,D | 50-2000 | 5-40 | Sparging argon | 50-150°C |
| Catalysts of Group 1 | 50-500 | 5-40 | Vacuum | 50-150°C |
| Ru catalysts of formula 3 | 50-500 | 5-40 | Vacuum | 50-150°C |
| Catalysts of formulas A,B,C,D | 50-500 | 5-40 | Vacuum | 50-150°C |
| Catalysts of Group 1 | 50-500 | 5-40 | Sparging inert gas | 50-150°C |
| Ru catalysts of formula 3 | 50-500 | 5-40 | Sparging inert gas | 50-150°C |
| Catalysts of formulas A,B,C,D | 50-500 | 5-40 | Sparging inert gas | 50-150°C |
| Catalysts of Group 1 | 50-500 | 5-40 | Sparging argon | 50-150°C |
| Ru catalysts of formula 3 | 50-500 | 5-40 | Sparging argon | 50-150°C |
| Catalysts of formulas A,B,C,D | 50-500 | 5-40 | Sparging argon | 50-150°C |
| Catalysts of Group 1 | 1-10000 | 8-40 | Vacuum | 50-150°C |
| Ru catalysts of formula 3 | 1-10000 | 8-40 | Vacuum | 50-150°C |
| Catalysts of formulas A,B,C,D | 1-10000 | 8-40 | Vacuum | 50-150°C |
| Catalysts of Group 1 | 1-10000 | 8-40 | Sparging inert gas | 50-150°C |
| Ru catalysts of formula 3 | 1-10000 | 8-40 | Sparging inert gas | 50-150°C |
| Catalysts of formulas A,B,C,D | 1-10000 | 8-40 | Sparging inert gas | 50-150°C |
| Catalysts of Group 1 | 1-10000 | 8-40 | Sparging argon | 50-150°C |
| Ru catalysts of formula 3 | 1-10000 | 8-40 | Sparging argon | 50-150°C |
| Catalysts of formulas A,B,C,D | 1-10000 | 8-40 | Sparging argon | 50-150°C |
| Catalysts of Group 1 | 50-2000 | 8-40 | Vacuum | 50-150°C |
| Ru catalysts of formula 3 | 50-2000 | 8-40 | Vacuum | 50-150°C |
| Catalysts of formulas A,B,C,D | 50-2000 | 8-40 | Vacuum | 50-150°C |
| Catalysts of Group 1 | 50-2000 | 8-40 | Sparging inert gas | 50-150°C |
| Ru catalysts of formula 3 | 50-2000 | 8-40 | Sparging inert gas | 50-150°C |
| Catalysts of formulas A,B,C,D | 50-2000 | 8-40 | Sparging inert gas | 50-150°C |
| Catalysts of Group 1 | 50-2000 | 8-40 | Sparging argon | 50-150°C |
| Ru catalysts of formula 3 | 50-2000 | 8-40 | Sparging argon | 50-150°C |
| Catalysts of formulas A,B,C,D | 50-2000 | 8-40 | Sparging argon | 50-150°C |
| Catalysts of Group 1 | 50-500 | 8-40 | Vacuum | 50-150°C |
| Ru catalysts of formula 3 | 50-500 | 8-40 | Vacuum | 50-150°C |
| Catalysts of formulas A,B,C,D | 50-500 | 8-40 | Vacuum | 50-150°C |
| Catalysts of Group 1 | 50-500 | 8-40 | Sparging inert gas | 50-150°C |
| Ru catalysts of formula 3 | 50-500 | 8-40 | Sparging inert gas | 50-150°C |
| Catalysts of formulas A,B,C,D | 50-500 | 8-40 | Sparging inert gas | 50-150°C |
| Catalysts of Group 1 | 50-500 | 8-40 | Sparging argon | 50-150°C |
| Ru catalysts of formula 3 | 50-500 | 8-40 | Sparging argon | 50-150°C |
| Catalysts of formulas A,B,C,D | 50-500 | 8-40 | Sparging argon | 50-150°C |
| Catalysts of Group 1 | 1-10000 | 8-20 | Vacuum | 50-150°C |
| Ru catalysts of formula 3 | 1-10000 | 8-20 | Vacuum | 50-150°C |
| Catalysts of formulas A,B,C,D | 1-10000 | 8-20 | Vacuum | 50-150°C |
| Catalysts of Group 1 | 1-10000 | 8-20 | Sparging inert gas | 50-150°C |
| Ru catalysts of formula 3 | 1-10000 | 8-20 | Sparging inert gas | 50-150°C |
| Catalysts of formulas A,B,C,D | 1-10000 | 8-20 | Sparging inert gas | 50-150°C |
| Catalysts of Group 1 | 1-10000 | 8-20 | Sparging argon | 50-150°C |
| Ru catalysts of formula 3 | 1-10000 | 8-20 | Sparging argon | 50-150°C |
| Catalysts of formulas A,B,C,D | 1-10000 | 8-20 | Sparging argon | 50-150°C |
| Catalysts of Group 1 | 50-2000 | 8-20 | Vacuum | 50-150°C |
| Ru catalysts of formula 3 | 50-2000 | 8-20 | Vacuum | 50-150°C |
| Catalysts of formulas A,B,C,D | 50-2000 | 8-20 | Vacuum | 50-150°C |
| Catalysts of Group 1 | 50-2000 | 8-20 | Sparging inert gas | 50-150°C |
| Ru catalysts of formula 3 | 50-2000 | 8-20 | Sparging inert gas | 50-150°C |
| Catalysts of formulas A,B,C,D | 50-2000 | 8-20 | Sparging inert gas | 50-150°C |
| Catalysts of Group 1 | 50-2000 | 8-20 | Sparging argon | 50-150°C |
| Ru catalysts of formula 3 | 50-2000 | 8-20 | Sparging argon | 50-150°C |
| Catalysts of formulas A,B,C,D | 50-2000 | 8-20 | Sparging argon | 50-150°C |
| Catalysts of Group 1 | 50-500 | 8-20 | Vacuum | 50-150°C |
| Ru catalysts of formula 3 | 50-500 | 8-20 | Vacuum | 50-150°C |
| Catalysts of formulas A,B,C,D | 50-500 | 8-20 | Vacuum | 50-150°C |
| Catalysts of Group 1 | 50-500 | 8-20 | Sparging inert gas | 50-150°C |
| Ru catalysts of formula 3 | 50-500 | 8-20 | Sparging inert gas | 50-150°C |
| Catalysts of formulas A,B,C,D | 50-500 | 8-20 | Sparging inert gas | 50-150°C |
| Catalysts of Group 1 | 50-500 | 8-20 | Sparging argon | 50-150°C |
| Ru catalysts of formula 3 | 50-500 | 8-20 | Sparging argon | 50-150°C |
| Catalysts of formulas A,B,C,D | 50-500 | 8-20 | Sparging argon | 50-150°C |
| Catalysts of Group 1 | 1-10000 | 0.2-4000 | Vacuum | 60-110°C |
| Ru catalysts of formula 3 | 1-10000 | 0.2-4000 | Vacuum | 60-110°C |
| Catalysts of formulas A,B,C,D | 1-10000 | 0.2-4000 | Vacuum | 60-110°C |
| Catalysts of Group 1 | 1-10000 | 0.2-4000 | Sparging inert gas | 60-110°C |
| Ru catalysts of formula 3 | 1-10000 | 0.2-4000 | Sparging inert gas | 60-110°C |
| Catalysts of formulas A,B,C,D | 1-10000 | 0.2-4000 | Sparging inert gas | 60-110°C |
| Catalysts of Group 1 | 1-10000 | 0.2-4000 | Sparging argon | 60-110°C |
| Ru catalysts of formula 3 | 1-10000 | 0.2-4000 | Sparging argon | 60-110°C |
| Catalysts of formulas A,B,C,D | 1-10000 | 0.2-4000 | Sparging argon | 60-110°C |
| Catalysts of Group 1 | 50-2000 | 0.2-4000 | Vacuum | 60-110°C |
| Ru catalysts of formula 3 | 50-2000 | 0.2-4000 | Vacuum | 60-110°C |
| Catalysts of formulas A,B,C,D | 50-2000 | 0.2-4000 | Vacuum | 60-110°C |
| Catalysts of Group 1 | 50-2000 | 0.2-4000 | Sparging inert gas | 60-110°C |
| Ru catalysts of formula 3 | 50-2000 | 0.2-4000 | Sparging inert gas | 60-110°C |
| Catalysts of formulas A,B,C,D | 50-2000 | 0.2-4000 | Sparging inert gas | 60-110°C |
| Catalysts of Group 1 | 50-2000 | 0.2-4000 | Sparging argon | 60-110°C |
| Ru catalysts of formula 3 | 50-2000 | 0.2-4000 | Sparging argon | 60-110°C |
| Catalysts of formulas A,B,C,D | 50-2000 | 0.2-4000 | Sparging argon | 60-110°C |
| Catalysts of Group 1 | 50-500 | 0.2-4000 | Vacuum | 60-110°C |
| Ru catalysts of formula 3 | 50-500 | 0.2-4000 | Vacuum | 60-110°C |
| Catalysts of formulas A,B,C,D | 50-500 | 0.2-4000 | Vacuum | 60-110°C |
| Catalysts of Group 1 | 50-500 | 0.2-4000 | Sparging inert gas | 60-110°C |
| Ru catalysts of formula 3 | 50-500 | 0.2-4000 | Sparging inert gas | 60-110°C |
| Catalysts of formulas A,B,C,D | 50-500 | 0.2-4000 | Sparging inert gas | 60-110°C |
| Catalysts of Group 1 | 50-500 | 0.2-4000 | Sparging argon | 60-110°C |
| Ru catalysts of formula 3 | 50-500 | 0.2-4000 | Sparging argon | 60-110°C |
| Catalysts of formulas A,B,C,D | 50-500 | 0.2-4000 | Sparging argon | 60-110°C |
| Catalysts of Group 1 | 1-10000 | 5-40 | Vacuum | 60-110°C |
| Ru catalysts of formula 3 | 1-10000 | 5-40 | Vacuum | 60-110°C |
| Catalysts of formulas A,B,C,D | 1-10000 | 5-40 | Vacuum | 60-110°C |
| Catalysts of Group 1 | 1-10000 | 5-40 | Sparging inert gas | 60-110°C |
| Ru catalysts of formula 3 | 1-10000 | 5-40 | Sparging inert gas | 60-110°C |
| Catalysts of formulas A,B,C,D | 1-10000 | 5-40 | Sparging inert gas | 60-110°C |
| Catalysts of Group 1 | 1-10000 | 5-40 | Sparging argon | 60-110°C |
| Ru catalysts of formula 3 | 1-10000 | 5-40 | Sparging argon | 60-110°C |
| Catalysts of formulas A,B,C,D | 1-10000 | 5-40 | Sparging argon | 60-110°C |
| Catalysts of Group 1 | 50-2000 | 5-40 | Vacuum | 60-110°C |
| Ru catalysts of formula 3 | 50-2000 | 5-40 | Vacuum | 60-110°C |
| Catalysts of formulas A,B,C,D | 50-2000 | 5-40 | Vacuum | 60-110°C |
| Catalysts of Group 1 | 50-2000 | 5-40 | Sparging inert gas | 60-110°C |
| Ru catalysts of formula 3 | 50-2000 | 5-40 | Sparging inert gas | 60-110°C |
| Catalysts of formulas A,B,C,D | 50-2000 | 5-40 | Sparging inert gas | 60-110°C |
| Catalysts of Group 1 | 50-2000 | 5-40 | Sparging argon | 60-110°C |
| Ru catalysts of formula 3 | 50-2000 | 5-40 | Sparging argon | 60-110°C |
| Catalysts of formulas A,B,C,D | 50-2000 | 5-40 | Sparging argon | 60-110°C |
| Catalysts of Group 1 | 50-500 | 5-40 | Vacuum | 60-110°C |
| Ru catalysts of formula 3 | 50-500 | 5-40 | Vacuum | 60-110°C |
| Catalysts of formulas A,B,C,D | 50-500 | 5-40 | Vacuum | 60-110°C |
| Catalysts of Group 1 | 50-500 | 5-40 | Sparging inert gas | 60-110°C |
| Ru catalysts of formula 3 | 50-500 | 5-40 | Sparging inert gas | 60-110°C |
| Catalysts of formulas A,B,C,D | 50-500 | 5-40 | Sparging inert gas | 60-110°C |
| Catalysts of Group 1 | 50-500 | 5-40 | Sparging argon | 60-110°C |
| Ru catalysts of formula 3 | 50-500 | 5-40 | Sparging argon | 60-110°C |
| Catalysts of formulas A,B,C,D | 50-500 | 5-40 | Sparging argon | 60-110°C |
| Catalysts of Group 1 | 1-10000 | 8-40 | Vacuum | 60-110°C |
| Ru catalysts of formula 3 | 1-10000 | 8-40 | Vacuum | 60-110°C |
| Catalysts of formulas A,B,C,D | 1-10000 | 8-40 | Vacuum | 60-110°C |
| Catalysts of Group 1 | 1-10000 | 8-40 | Sparging inert gas | 60-110°C |
| Ru catalysts of formula 3 | 1-10000 | 8-40 | Sparging inert gas | 60-110°C |
| Catalysts of formulas A,B,C,D | 1-10000 | 8-40 | Sparging inert gas | 60-110°C |
| Catalysts of Group 1 | 1-10000 | 8-40 | Sparging argon | 60-110°C |
| Ru catalysts of formula 3 | 1-10000 | 8-40 | Sparging argon | 60-110°C |
| Catalysts of formulas A,B,C,D | 1-10000 | 8-40 | Sparging argon | 60-110°C |
| Catalysts of Group 1 | 50-2000 | 8-40 | Vacuum | 60-110°C |
| Ru catalysts of formula 3 | 50-2000 | 8-40 | Vacuum | 60-110°C |
| Catalysts of formulas A,B,C,D | 50-2000 | 8-40 | Vacuum | 60-110°C |
| Catalysts of Group 1 | 50-2000 | 8-40 | Sparging inert gas | 60-110°C |
| Ru catalysts of formula 3 | 50-2000 | 8-40 | Sparging inert gas | 60-110°C |
| Catalysts of formulas A,B,C,D | 50-2000 | 8-40 | Sparging inert gas | 60-110°C |
| Catalysts of Group 1 | 50-2000 | 8-40 | Sparging argon | 60-110°C |
| Ru catalysts of formula 3 | 50-2000 | 8-40 | Sparging argon | 60-110°C |
| Catalysts of formulas A,B,C,D | 50-2000 | 8-40 | Sparging argon | 60-110°C |
| Catalysts of Group 1 | 50-500 | 8-40 | Vacuum | 60-110°C |
| Ru catalysts of formula 3 | 50-500 | 8-40 | Vacuum | 60-110°C |
| Catalysts of formulas A,B,C,D | 50-500 | 8-40 | Vacuum | 60-110°C |
| Catalysts of Group 1 | 50-500 | 8-40 | Sparging inert gas | 60-110°C |
| Ru catalysts of formula 3 | 50-500 | 8-40 | Sparging inert gas | 60-110°C |
| Catalysts of formulas A,B,C,D | 50-500 | 8-40 | Sparging inert gas | 60-110°C |
| Catalysts of Group 1 | 50-500 | 8-40 | Sparging argon | 60-110°C |
| Ru catalysts of formula 3 | 50-500 | 8-40 | Sparging argon | 60-110°C |
| Catalysts of formulas A,B,C,D | 50-500 | 8-40 | Sparging argon | 60-110°C |
| Catalysts of Group 1 | 1-10000 | 8-20 | Vacuum | 60-110°C |
| Ru catalysts of formula 3 | 1-10000 | 8-20 | Vacuum | 60-110°C |
| Catalysts of formulas A,B,C,D | 1-10000 | 8-20 | Vacuum | 60-110°C |
| Catalysts of Group 1 | 1-10000 | 8-20 | Sparging inert gas | 60-110°C |
| Ru catalysts of formula 3 | 1-10000 | 8-20 | Sparging inert gas | 60-110°C |
| Catalysts of formulas A,B,C,D | 1-10000 | 8-20 | Sparging inert gas | 60-110°C |
| Catalysts of Group 1 | 1-10000 | 8-20 | Sparging argon | 60-110°C |
| Ru catalysts of formula 3 | 1-10000 | 8-20 | Sparging argon | 60-110°C |
| Catalysts of formulas A,B,C,D | 1-10000 | 8-20 | Sparging argon | 60-110°C |
| Catalysts of Group 1 | 50-2000 | 8-20 | Vacuum | 60-110°C |
| Ru catalysts of formula 3 | 50-2000 | 8-20 | Vacuum | 60-110°C |
| Catalysts of formulas A,B,C,D | 50-2000 | 8-20 | Vacuum | 60-110°C |
| Catalysts of Group 1 | 50-2000 | 8-20 | Sparging inert gas | 60-110°C |
| Ru catalysts of formula 3 | 50-2000 | 8-20 | Sparging inert gas | 60-110°C |
| Catalysts of formulas A,B,C,D | 50-2000 | 8-20 | Sparging inert gas | 60-110°C |
| Catalysts of Group 1 | 50-2000 | 8-20 | Sparging argon | 60-110°C |
| Ru catalysts of formula 3 | 50-2000 | 8-20 | Sparging argon | 60-110°C |
| Catalysts of formulas A,B,C,D | 50-2000 | 8-20 | Sparging argon | 60-110°C |
| Catalysts of Group 1 | 50-500 | 8-20 | Vacuum | 60-110°C |
| Ru catalysts of formula 3 | 50-500 | 8-20 | Vacuum | 60-110°C |
| Catalysts of formulas A,B,C,D | 50-500 | 8-20 | Vacuum | 60-110°C |
| Catalysts of Group 1 | 50-500 | 8-20 | Sparging inert gas | 60-110°C |
| Ru catalysts of formula 3 | 50-500 | 8-20 | Sparging inert gas | 60-110°C |
| Catalysts of formulas A,B,C,D | 50-500 | 8-20 | Sparging inert gas | 60-110°C |
| Catalysts of Group 1 | 50-500 | 8-20 | Sparging argon | 60-110°C |
| Ru catalysts of formula 3 | 50-500 | 8-20 | Sparging argon | 60-110°C |
| Catalysts of formulas A,B,C,D | 50-500 | 8-20 | Sparging argon | 60-110°C |
| Catalysts of Group 1 | 1-10000 | 0.2-4000 | Vacuum | 80°C |
| Ru catalysts of formula 3 | 1-10000 | 0.2-4000 | Vacuum | 80°C |
| Catalysts of formulas A,B,C,D | 1-10000 | 0.2-4000 | Vacuum | 80°C |
| Catalysts of Group 1 | 1-10000 | 0.2-4000 | Sparging inert gas | 80°C |
| Ru catalysts of formula 3 | 1-10000 | 0.2-4000 | Sparging inert gas | 80°C |
| Catalysts of formulas A,B,C,D | 1-10000 | 0.2-4000 | Sparging inert gas | 80°C |
| Catalysts of Group 1 | 1-10000 | 0.2-4000 | Sparging argon | 80°C |
| Ru catalysts of formula 3 | 1-10000 | 0.2-4000 | Sparging argon | 80°C |
| Catalysts of formulas A,B,C,D | 1-10000 | 0.2-4000 | Sparging argon | 80°C |
| Catalysts of Group 1 | 50-2000 | 0.2-4000 | Vacuum | 80°C |
| Ru catalysts of formula 3 | 50-2000 | 0.2-4000 | Vacuum | 80°C |
| Catalysts of formulas A,B,C,D | 50-2000 | 0.2-4000 | Vacuum | 80°C |
| Catalysts of Group 1 | 50-2000 | 0.2-4000 | Sparging inert gas | 80°C |
| Ru catalysts of formula 3 | 50-2000 | 0.2-4000 | Sparging inert gas | 80°C |
| Catalysts of formulas A,B,C,D | 50-2000 | 0.2-4000 | Sparging inert gas | 80°C |
| Catalysts of Group 1 | 50-2000 | 0.2-4000 | Sparging argon | 80°C |
| Ru catalysts of formula 3 | 50-2000 | 0.2-4000 | Sparging argon | 80°C |
| Catalysts of formulas A,B,C,D | 50-2000 | 0.2-4000 | Sparging argon | 80°C |
| Catalysts of Group 1 | 50-500 | 0.2-4000 | Vacuum | 80°C |
| Ru catalysts of formula 3 | 50-500 | 0.2-4000 | Vacuum | 80°C |
| Catalysts of formulas A,B,C,D | 50-500 | 0.2-4000 | Vacuum | 80°C |
| Catalysts of Group 1 | 50-500 | 0.2-4000 | Sparging inert gas | 80°C |
| Ru catalysts of formula 3 | 50-500 | 0.2-4000 | Sparging inert gas | 80°C |
| Catalysts of formulas A,B,C,D | 50-500 | 0.2-4000 | Sparging inert gas | 80°C |
| Catalysts of Group 1 | 50-500 | 0.2-4000 | Sparging argon | 80°C |
| Ru catalysts of formula 3 | 50-500 | 0.2-4000 | Sparging argon | 80°C |
| Catalysts of formulas A,B,C,D | 50-500 | 0.2-4000 | Sparging argon | 80°C |
| Catalysts of Group 1 | 1-10000 | 5-40 | Vacuum | 80°C |
| Ru catalysts of formula 3 | 1-10000 | 5-40 | Vacuum | 80°C |
| Catalysts of formulas A,B,C,D | 1-10000 | 5-40 | Vacuum | 80°C |
| Catalysts of Group 1 | 1-10000 | 5-40 | Sparging inert gas | 80°C |
| Ru catalysts of formula 3 | 1-10000 | 5-40 | Sparging inert gas | 80°C |
| Catalysts of formulas A,B,C,D | 1-10000 | 5-40 | Sparging inert gas | 80°C |
| Catalysts of Group 1 | 1-10000 | 5-40 | Sparging argon | 80°C |
| Ru catalysts of formula 3 | 1-10000 | 5-40 | Sparging argon | 80°C |
| Catalysts of formulas A,B,C,D | 1-10000 | 5-40 | Sparging argon | 80°C |
| Catalysts of Group 1 | 50-2000 | 5-40 | Vacuum | 80°C |
| Ru catalysts of formula 3 | 50-2000 | 5-40 | Vacuum | 80°C |
| Catalysts of formulas A,B,C,D | 50-2000 | 5-40 | Vacuum | 80°C |
| Catalysts of Group 1 | 50-2000 | 5-40 | Sparging inert gas | 80°C |
| Ru catalysts of formula 3 | 50-2000 | 5-40 | Sparging inert gas | 80°C |
| Catalysts of formulas A,B,C,D | 50-2000 | 5-40 | Sparging inert gas | 80°C |
| Catalysts of Group 1 | 50-2000 | 5-40 | Sparging argon | 80°C |
| Ru catalysts of formula 3 | 50-2000 | 5-40 | Sparging argon | 80°C |
| Catalysts of formulas A,B,C,D | 50-2000 | 5-40 | Sparging argon | 80°C |
| Catalysts of Group 1 | 50-500 | 5-40 | Vacuum | 80°C |
| Ru catalysts of formula 3 | 50-500 | 5-40 | Vacuum | 80°C |
| Catalysts of formulas A,B,C,D | 50-500 | 5-40 | Vacuum | 80°C |
| Catalysts of Group 1 | 50-500 | 5-40 | Sparging inert gas | 80°C |
| Ru catalysts of formula 3 | 50-500 | 5-40 | Sparging inert gas | 80°C |
| Catalysts of formulas A,B,C,D | 50-500 | 5-40 | Sparging inert gas | 80°C |
| Catalysts of Group 1 | 50-500 | 5-40 | Sparging argon | 80°C |
| Ru catalysts of formula 3 | 50-500 | 5-40 | Sparging argon | 80°C |
| Catalysts of formulas A,B,C,D | 50-500 | 5-40 | Sparging argon | 80°C |
| Catalysts of Group 1 | 1-10000 | 8-40 | Vacuum | 80°C |
| Ru catalysts of formula 3 | 1-10000 | 8-40 | Vacuum | 80°C |
| Catalysts of formulas A,B,C,D | 1-10000 | 8-40 | Vacuum | 80°C |
| Catalysts of Group 1 | 1-10000 | 8-40 | Sparging inert gas | 80°C |
| Ru catalysts of formula 3 | 1-10000 | 8-40 | Sparging inert gas | 80°C |
| Catalysts of formulas A,B,C,D | 1-10000 | 8-40 | Sparging inert gas | 80°C |
| Catalysts of Group 1 | 1-10000 | 8-40 | Sparging argon | 80°C |
| Ru catalysts of formula 3 | 1-10000 | 8-40 | Sparging argon | 80°C |
| Catalysts of formulas A,B,C,D | 1-10000 | 8-40 | Sparging argon | 80°C |
| Catalysts of Group 1 | 50-2000 | 8-40 | Vacuum | 80°C |
| Ru catalysts of formula 3 | 50-2000 | 8-40 | Vacuum | 80°C |
| Catalysts of formulas A,B,C,D | 50-2000 | 8-40 | Vacuum | 80°C |
| Catalysts of Group 1 | 50-2000 | 8-40 | Sparging inert gas | 80°C |
| Ru catalysts of formula 3 | 50-2000 | 8-40 | Sparging inert gas | 80°C |
| Catalysts of formulas A,B,C,D | 50-2000 | 8-40 | Sparging inert gas | 80°C |
| Catalysts of Group 1 | 50-2000 | 8-40 | Sparging argon | 80°C |
| Ru catalysts of formula 3 | 50-2000 | 8-40 | Sparging argon | 80°C |
| Catalysts of formulas A,B,C,D | 50-2000 | 8-40 | Sparging argon | 80°C |
| Catalysts of Group 1 | 50-500 | 8-40 | Vacuum | 80°C |
| Ru catalysts of formula 3 | 50-500 | 8-40 | Vacuum | 80°C |
| Catalysts of formulas A,B,C,D | 50-500 | 8-40 | Vacuum | 80°C |
| Catalysts of Group 1 | 50-500 | 8-40 | Sparging inert gas | 80°C |
| Ru catalysts of formula 3 | 50-500 | 8-40 | Sparging inert gas | 80°C |
| Catalysts of formulas A,B,C,D | 50-500 | 8-40 | Sparging inert gas | 80°C |
| Catalysts of Group 1 | 50-500 | 8-40 | Sparging argon | 80°C |
| Ru catalysts of formula 3 | 50-500 | 8-40 | Sparging argon | 80°C |
| Catalysts of formulas A,B,C,D | 50-500 | 8-40 | Sparging argon | 80°C |
| Catalysts of Group 1 | 1-10000 | 8-20 | Vacuum | 80°C |
| Ru catalysts of formula 3 | 1-10000 | 8-20 | Vacuum | 80°C |
| Catalysts of formulas A,B,C,D | 1-10000 | 8-20 | Vacuum | 80°C |
| Catalysts of Group 1 | 1-10000 | 8-20 | Sparging inert gas | 80°C |
| Ru catalysts of formula 3 | 1-10000 | 8-20 | Sparging inert gas | 80°C |
| Catalysts of formulas A,B,C,D | 1-10000 | 8-20 | Sparging inert gas | 80°C |
| Catalysts of Group 1 | 1-10000 | 8-20 | Sparging argon | 80°C |
| Ru catalysts of formula 3 | 1-10000 | 8-20 | Sparging argon | 80°C |
| Catalysts of formulas A,B,C,D | 1-10000 | 8-20 | Sparging argon | 80°C |
| Catalysts of Group 1 | 50-2000 | 8-20 | Vacuum | 80°C |
| Ru catalysts of formula 3 | 50-2000 | 8-20 | Vacuum | 80°C |
| Catalysts of formulas A,B,C,D | 50-2000 | 8-20 | Vacuum | 80°C |
| Catalysts of Group 1 | 50-2000 | 8-20 | Sparging inert gas | 80°C |
| Ru catalysts of formula 3 | 50-2000 | 8-20 | Sparging inert gas | 80°C |
| Catalysts of formulas A,B,C,D | 50-2000 | 8-20 | Sparging inert gas | 80°C |
| Catalysts of Group 1 | 50-2000 | 8-20 | Sparging argon | 80°C |
| Ru catalysts of formula 3 | 50-2000 | 8-20 | Sparging argon | 80°C |
| Catalysts of formulas A,B,C,D | 50-2000 | 8-20 | Sparging argon | 80°C |
| Catalysts of Group 1 | 50-500 | 8-20 | Vacuum | 80°C |
| Ru catalysts of formula 3 | 50-500 | 8-20 | Vacuum | 80°C |
| Catalysts of formulas A,B,C,D | 50-500 | 8-20 | Vacuum | 80°C |
| Catalysts of Group 1 | 50-500 | 8-20 | Sparging inert gas | 80°C |
| Ru catalysts of formula 3 | 50-500 | 8-20 | Sparging inert gas | 80°C |
| Catalysts of formulas A,B,C,D | 50-500 | 8-20 | Sparging inert gas | 80°C |
| Catalysts of Group 1 | 50-500 | 8-20 | Sparging argon | 80°C |
| Ru catalysts of formula 3 | 50-500 | 8-20 | Sparging argon | 80°C |
| Catalysts of formulas A,B,C,D | 50-500 | 8-20 | Sparging argon | 80°C |

### Experimental Section

Practically, the reactions were carried out in round bottomed flasks fitted with an effective reflux condenser and placed in an oil bath. Inert gas sparging was provided by a vigorous argon stream through a long thin needle introduced through a rubber septum. Stirring at high rate gives rise to better dispersion of the gas bubbles and therefore increases the speed of the gas transfer.
The results of the catalytic runs can be rather sensitive to impurities present in the substrates when working with ultralow catalyst loadings. Therefore, all starting dienes were prepared by careful purification using repeated vacuum distillation or recrystallization.
Toluene was purified using recommended methods, then dried over 3Å molecular sieves, thereby lowering moisture content to 1-2 ppm, and degassed by ultrasonication.
In order to evaluate the catalytic performance in toluene solutions, RCM reactions were performed at 80°C and concentrations from 8 to 40 mM in the presence of alkanes (dodecane, tetradecane and octadecane) as internal standards. The course of the reaction was monitored by GC analysis taking aliquots with syringes and quenching in ethyl vinyl ether solution.

As metathesis catalysts ruthenium catalysts of formulas A-D have been applied.

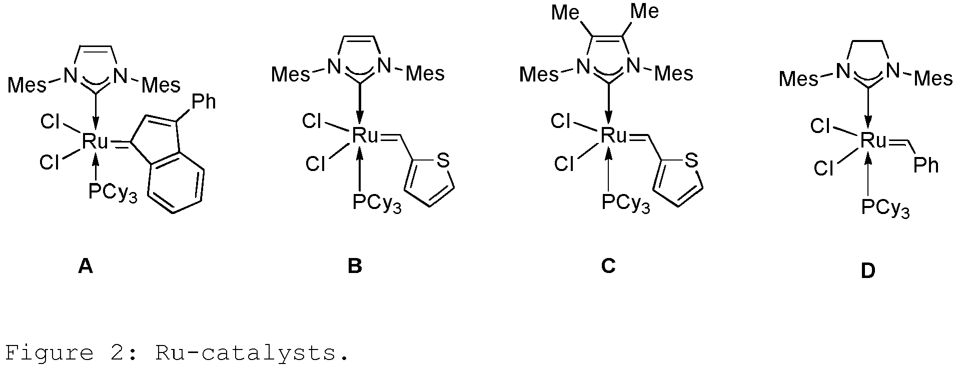

### Examples of Metathesis Reactions

**Table 2: Identified products in metathesis of olefins.**

| Olefin | Products | |
|---|---|---|
| 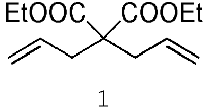 | 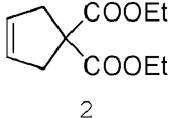 | |
| 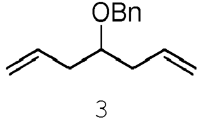 | 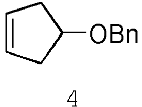 | |
| 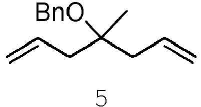 | 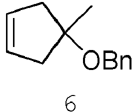 | |
| 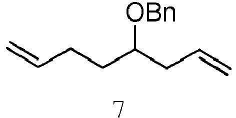 | 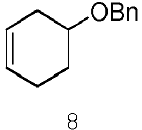 | |
| 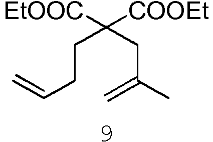 | 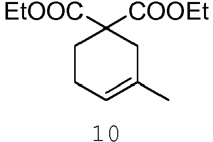 | |
| 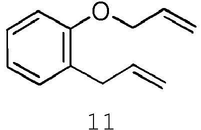 | 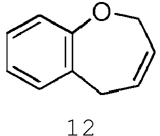 | |
| 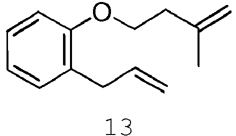 | 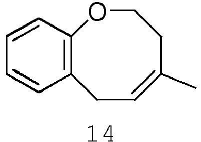 | 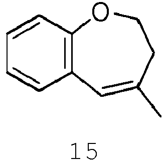 |
| | 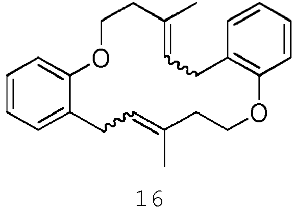 | |

**Table 3: Metathesis of olefins 1, 3, 5, 7, 9, 11 and 13 in toluene at 80°C according to procedure B.**

| Olefin | mM [a] | Catalyst | ppm [b] | TOF [c] | Conv. [d] |
|---|---|---|---|---|---|
| **1** | 40 | B | 50 | 2063 | 95 |
| **1** | 40 | C | 50 | 4173 | 95 |
| **1** | 40 | D | 50 | 2984 | 92 |
| **3** | 20 | A | 100 | 122 | 77 |
| **3** | 20 | B | 100 | 372 | 52 |
| **3** | 20 | C | 100 | 826 | 79 |
| **5** | 20 | A | 50 | 199 | 78 |
| **5** | 20 | B | 50 | 810 | 69 |
| **5** | 20 | C | 50 | 1762 | 78 |
| **7** | 20 | A | 50 | 85 | 65 |
| **7** | 20 | B | 50 | 572 | 57 |
| **7** | 20 | C | 50 | 1281 | 85 |
| **9** | 40 | A | 50 | 56 | 54 |
| **9** | 40 | B | 50 | 1285 | 76 |
| **9** | 40 | C | 50 | 1711 | 80 |
| **11** | 20 | A | 100 | 394 | 98 |
| **11** | 20 | B | 100 | 1048 | 81 |
| **11** | 20 | C | 100 | 1380 | 90 |
| **13** | 20 | A | 500 | 36 | 94 |
| **13** | 20 | B | 500 | 40 | 92 |
| **13** | 20 | C | 500 | 87 | 96 |

| | | | | | |
|---|---|---|---|---|---|
| [a] starting concentration of olefin; [b] catalyst loading 1 ppm = 0.0001 mol%; [c] turn-over frequency [min⁻¹]; [d] conversion of starting olefin measured after complete standstill of the reaction [%]. | | | | | |

**Table 4: Isolated products in metathesis of protected amines.**

| Olefin | Products | |
|---|---|---|
| 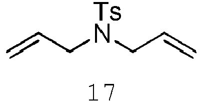 | 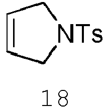 | |
| 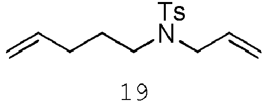 | 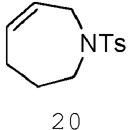 | |
| 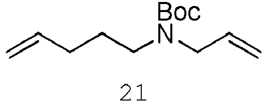 | 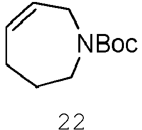 | |
| 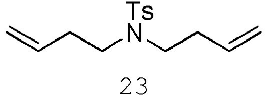 | 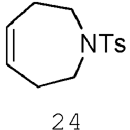 | 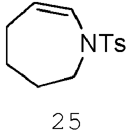 |
| 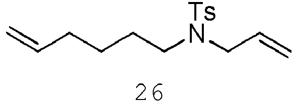 | 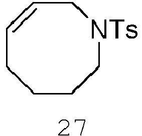 | |
| 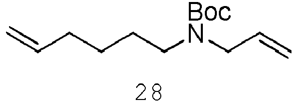 | 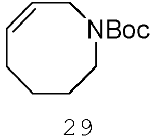 | 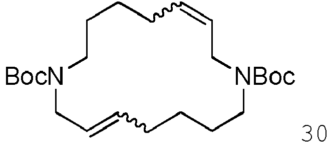 |
| 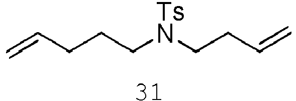 | 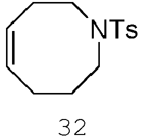 | |
| 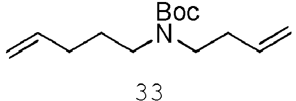 | 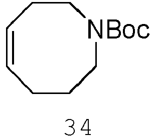 | |

**Table 5: Metathesis of protected amines in toluene at 80°C according to procedure B.**

| Olefin | mM [a] | Catalyst | ppm [b] | TOF [c] | Conv. [d] |
|---|---|---|---|---|---|
| **17** | 20 | A | 50 | 277 | 59 |
| **17** | 20 | B | 50 | 1010 | 60 |
| **17** | 20 | C | 50 | 1820 | 54 |
| **17** | 200 | C | 100 | 1700 | 88 |
| **19** | 20 | A | 50 | 764 | 99 |
| **19** | 20 | B | 50 | 1043 | 68 |
| **19** | 20 | B | 100 | 900 | 96 |
| **19** | 20 | C | 50 | 1152 | 62 |
| **19** | 20 | C | +50 | 541 | 92 |
| **21** | 20 | A | 200 | 55 | 74 |
| **21** | 20 | B | 200 | 129 | 72 |
| **21** | 20 | C | 200 | 150 | 74 |
| **23** | 20 | A | 100 | 188 | 82 |
| **23** | 20 | B | 100 | 433 | 66 |
| **23** | 20 | C | 100 | 586 | 81 |
| **23** | 20 | C | +100 | 113 | 97 |
| **26** | 8 | A | 250 | 52 | 60 |
| **26** | 8 | B | 500 | 109 | 79 |
| **26** | 8 | C | 250 | 179 | 65 |
| **28** | 20 | A | 500 | 67 | 95 |
| **28** | 20 | B | 500 | 127 | 83 |
| **28** | 20 | C | 500 | 152 | 92 |
| **33** | 8 | A | 200 | 79 | 100 |
| **33** | 8 | B | 150 | 478 | 90 |
| **33** | 8 | C | 200 | 357 | 99 |

| | | | | | |
|---|---|---|---|---|---|
| [a] starting concentration of olefin;.[b] catalyst loading 1 ppm = 0.0001 mol%, + denotes additional amounts of catalyst added after stopping of the reaction; [c] turn-over frequency [min⁻¹]; [d] conversion of starting olefin measured after complete standstill of the reaction [%]. | | | | | |

**Table 6: Metathesis of 2-allylphenol esters.**

| Olefin | Products | |
|---|---|---|
| 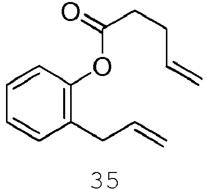 | 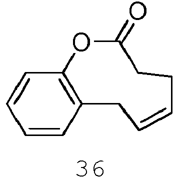 | 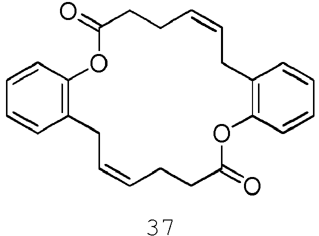 |
| 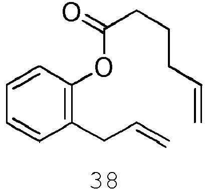 | 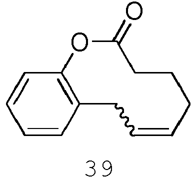 | 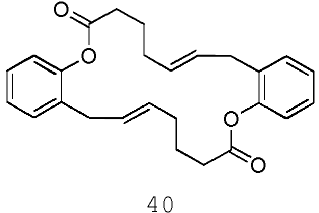 |
| 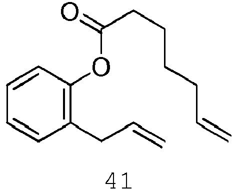 | 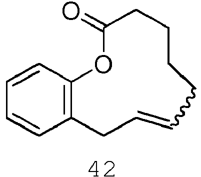 | 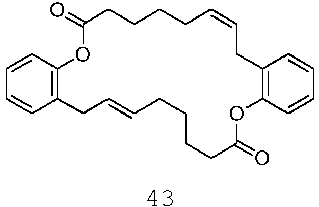 |
| 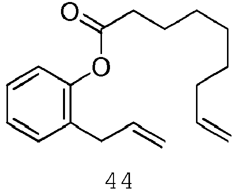 | 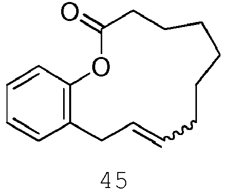 | 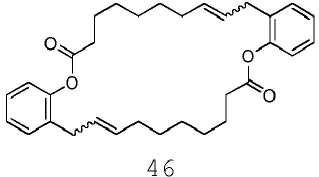 |
| 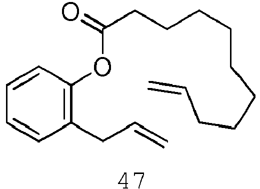 | 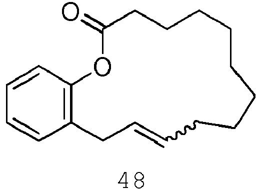 | 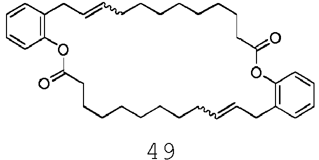 |
| | | 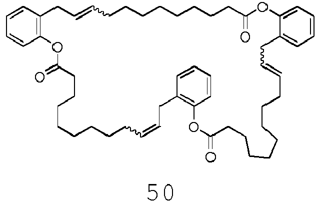 |
| 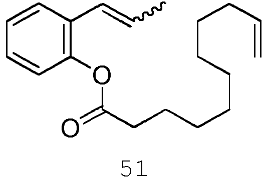 | 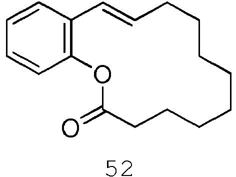 | 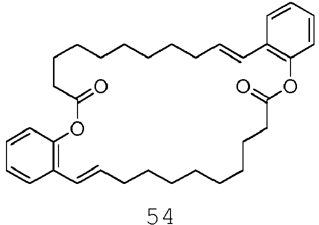 |
| | 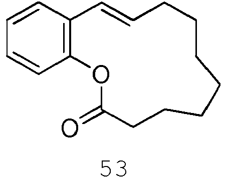 | |

**Table 7: Metathesis of 2-allylphenol esters in toluene at 80°C according to procedure B.**

| Olefin | mM [a] | Catalyst | ppm [b] | TOF [c] | Conv. [d] |
|---|---|---|---|---|---|
| **35** | 20 | A | 100 | 267 | 82 |
| **35** | 20 | B | 100 | 761 | 68 |
| **35** | 20 | C | 100 | 1100 | 89 |
| **38** | 20 | B | 50 | 1026 | 64 |
| **38** | 8 | C | 200 | 474 | 99 |
| **41** | 8 | B | 100 | 656 | 76 |
| **41** | 8 | C | 100 | 1381 | 72 |
| **44** | 8 | B | 150 | 471 | 98 |
| **44** | 20 | B | 100 | 753 | 96 |
| **44** | 8 | C | 150 | 1047 | 98 |
| **47** | 8 | A | 100 | 36 | 88 |
| **47** | 8 | B | 150 | 446 | 91 |
| **47** | 8 | C | 100 | 1026 | 70 |
| **47** | 8 | C | +100 | 365 | 100 |
| **51** | 8 | A | 500 | 35 | 93 |
| **51** | 8 | B | 500 | 76 | 80 |
| **51** | 8 | C | 500 | 107 | 84 |

| | | | | | |
|---|---|---|---|---|---|
| [a] starting concentration of olefin;.[b] catalyst loading 1 ppm = 0.0001 mol%, + denotes additional amounts of catalyst added after stopping of the reaction; [c] turn-over frequency [min⁻¹]; [d] conversion of starting olefin measured after complete standstill of the reaction [%]. | | | | | |

**Table 8: Metathesis of 1,ω-dienyl esters.**

| Olefin | Products | |
|---|---|---|
| 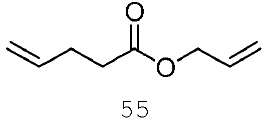 | 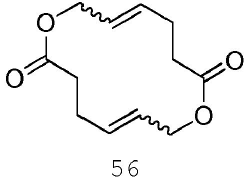 | 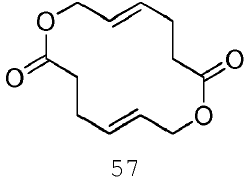 |
| 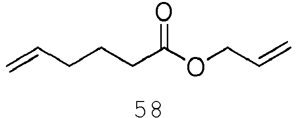 | 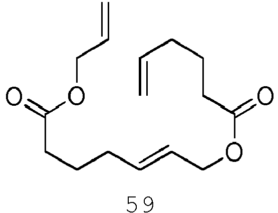 | 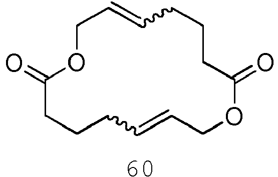 |
| 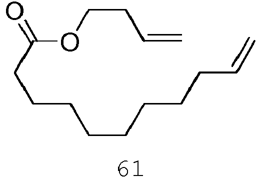 | 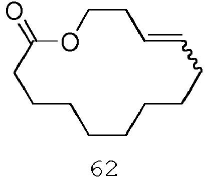 | 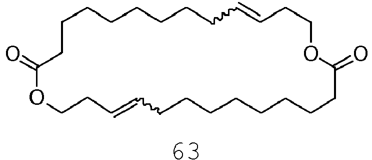 |
| 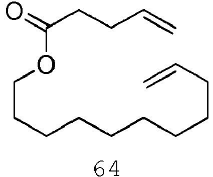 | 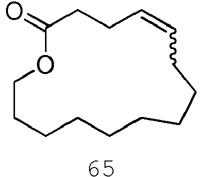 | 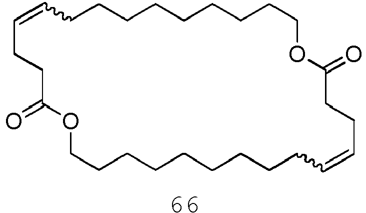 |
| 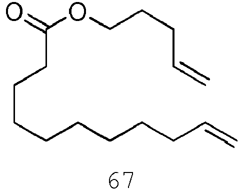 | 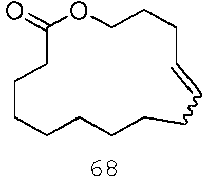 | 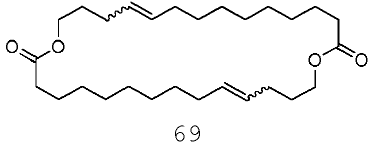 |
| 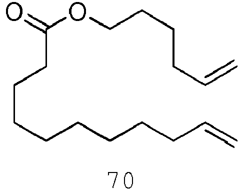 | 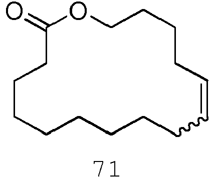 | 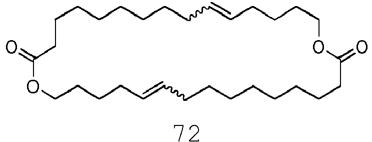 |

**Table 9: Metathesis of 1,ω-dienyl esters in toluene at 80°C according to procedure B.**

| Olefin | mM [a] | Catalyst | ppm [b] | TOF [c] | Conv. [d] |
|---|---|---|---|---|---|
| **61** | 40 | A | 50 | 278 | 100 |
| **61** | 10 | B | 50 | 739 | 100 |
| **61** | 40 | B | 50 | 1022 | 95 |
| **61** | 20 | C | 50 | 1049 | 86 |
| **61** | 40 | C | 50 | 1495 | 88 |
| **61** | 400 | C | 50 | 2422 | 89 |
| **64** | 10 | A | 75 | 105 | 84 |
| **64** | 10 | B | 75 | 321 | 92 |
| **64** | 10 | C | 75 | 430 | 98 |
| **67** | 8 | B | 100 | 118 | 40 |
| **67** | 8 | C | 100 | 280 | 58 |
| **67** | 20 | C | 250 | 356 | 100 |
| **70** | 8 | A | 75 | 542 | 100 |
| **70** | 40 | A | 50 | 115 | 99 |
| **70** | 8 | B | 50 | 1014 | 92 |
| **70** | 8 | C | 75 | 2265 | 100 |

| | | | | | |
|---|---|---|---|---|---|
| [a] starting concentration of olefin;.[b] catalyst loading 1 ppm = 0.0001 mol%; [c] turn-over frequency [min-¹]; [d] conversion of starting olefin measured after complete standstill of the reaction [%]. | | | | | |

**Table 10: Metathesis of prolines**

| Olefin | Products | |
|---|---|---|
| 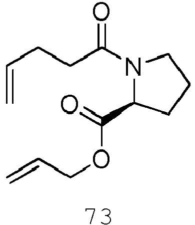 | 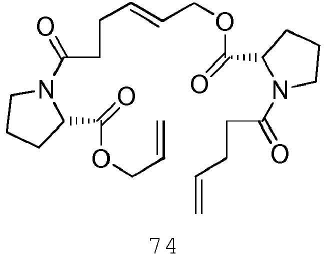 | 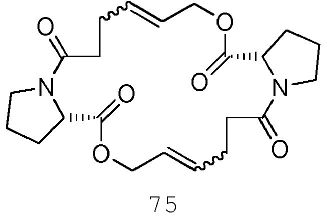 |
| 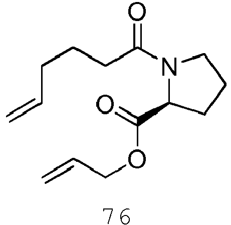 | 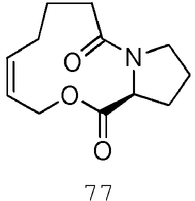 | 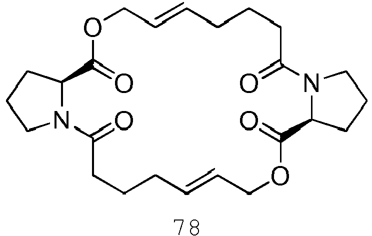 |
| 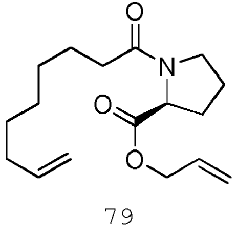 | 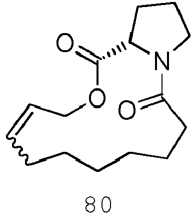 | 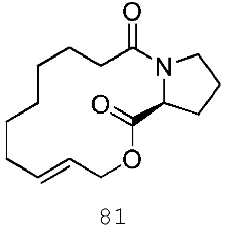 |
| 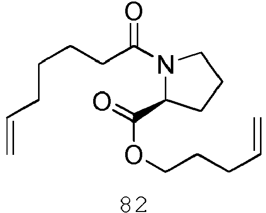 | 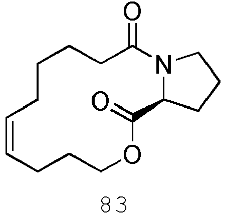 | 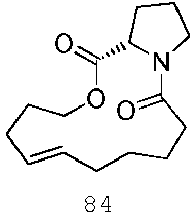 |
| 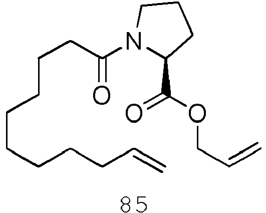 | 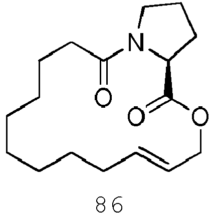 | |
| 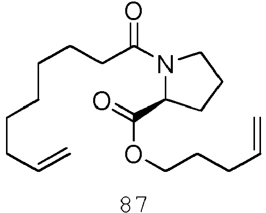 | 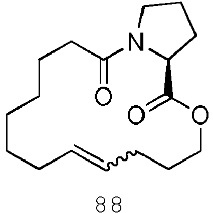 | |

**Table 11: Metathesis of prolines in toluene at 80°C according to procedure B.**

| Olefin | mM [a] | Catalyst | ppm [b] | TOF [c] | Conv. [d] |
|---|---|---|---|---|---|
| **76** | 8 | A | 1000 | 67 | 85 |
| **76** | 8 | B | 2000 | 30 | 70 |
| **76** | 8 | C | 1000 | 66 | 75 |
| **79** | 8 | A | 1000 | 14 | 80 |
| **79** | 8 | B | 1000 | 32 | 50 |
| **79** | 8 | B | +500 | 31 | 74 |
| **79** | 8 | C | 1000 | 43 | 58 |
| **79** | 8 | C | +500 | 32 | 79 |
| **82** | 8 | A | 200 | 126 | 91 |
| **82** | 8 | B | 200 | 261 | 80 |
| **82** | 8 | B | +100 | 102 | 94 |
| **82** | 8 | C | 200 | 375 | 91 |
| **85** | 8 | A | 1000 | 26 | 95 |
| **85** | 8 | B | 1000 | 52 | 80 |
| **85** | 8 | B | +500 | 15 | 92 |
| **85** | 8 | C | 1000 | 61 | 74 |
| **85** | 8 | C | +500 | 21 | 88 |
| **87** | 8 | A | 200 | 119 | 90 |
| **87** | 8 | B | 200 | 259 | 80 |
| **87** | 8 | B | +200 | 63 | 99 |
| **87** | 8 | C | 200 | 344 | 82 |
| **87** | 8 | C | +200 | 56 | 98 |

| | | | | | |
|---|---|---|---|---|---|
| [a] starting concentration of olefin;.[b] catalyst loading 1 ppm = 0.0001 mol%, + denotes additional amounts of catalyst added after stopping of the reaction; [c] turn-over frequency [min⁻¹]; [d] conversion of starting olefin measured after complete standstill of the reaction [%]. | | | | | |

### Experimental Procedures

### Procedure for metathesis of neat dienes (A)

A 250 ml round bottomed flask equipped with reflux condenser closed with oil bubbler, 3 cm magnetic stir bar and two rubber septa was flame-dried under vacuum, charged with substrate (0.2 mol), then filled with argon and the flask was placed in an oil bath heated to 80°C. Stirring (800 rpm) was started and argon was passed through a needle inserted through the septum and connected to a gas supply. After 30 min temperature equilibration a solution of catalyst in 0.8 ml of degassed toluene were added using a syringe. Samples were taken at regular intervals via syringe through the septum, quenched with 100 µl ethylvinylether in 1 ml toluene and analyzed using GC.

### Procedure for metathesis in diluted solution (B)

Toluene was degassed 10 min in an ultrasonic bath under argon prior to use. A 500 ml round bottomed flask equipped with intensive reflux condenser (50 cm) closed with oil bubbler, 3 cm magnetic stir bar and two rubber septa was flame-dried under vacuum, charged with substrate and internal standard (dodecane, tetradecane or octadecane was added as internal standard) then filled with argon and 250 ml of degassed toluene were added through steel capillary under argon. The flask was placed in an oil bath heated to 80°C, stirring (800 rpm) was started and argon was passed through a needle inserted through the septum and connected to a gas supply (100-300 ml/min). After 30 min temperature equilibration a solution of catalyst in 0.8 ml of degassed toluene were added using a syringe. Samples were taken at regular intervals via syringe through the septum, quenched with 50 µl ethylvinylether in 0.5 ml toluene and analyzed using GC.

### Metathesis diethyl 2,2-diallylmalonate (1)

Metathesis of neat diene 1 (48 g, 200 mmol) according to general procedure A using 0.01 mol% of catalyst B as a **solution** in toluene afforded complete conversion and 41 g (97%) of **diethyl 3-cyclopentene-1,1-dicarboxylate (2)** were isolated by distillation of reaction mixture under vacuum using a 10 cm Vigreux column; bp = 82°C/2mbar.

### Metathesis of 4-benzyloxy-hepta-1,6-diene (3)

Metathesis of neat diene **3** (40.46 g, 200 mmol) according to general procedure A using 0.02 mol% catalyst C as a solution in toluene afforded complete conversion and 33.4 g (96%) of **3-benzyloxy-cyclopentene-1 (4)** were isolated by distillation of reaction mixture under vacuum using a 10 cm Vigreux column; bp 74°C/0.8 mbar.

### Metathesis 4-benzyloxy-4-methyl-1,6-heptadiene (5)

Metathesis of neat diene **5** (43.26 g, 0.2 mol) according to general procedure A using 0.01 mol% catalyst C as a solution in toluene afforded complete conversion and 36.5 g (97%) of **3-benzyloxy-3-methyl-cyclopentene-1 (6)** were isolated by distillation under vacuum using a 5 cm Vigreux column; bp 66-68°C/0.7mbar.

### Metathesis of 4-benzyloxy-1,7-octadiene (7)

Metathesis of neat diene **7** (43.26 g, 0.2 mol) according to general procedure A using 0.01 mol% catalyst C as a solution in toluene afforded complete conversion and 35.8 g (95%) of **4-benzyloxy-1-cyclohexene (8)** were isolated by distillation of reaction mixture under vacuum using a 10 cm Vigreux column; bp = 71-73°C/0.6 mbar.

## Claims

1. Method for producing metathesis products comprising contacting metathesis starting materials under metathesis conditions with a metathesis catalyst, wherein the metathesis catalyst is employed in an amount of from 0.0001 mol-% to 1 mol-% based on half of the sum of the reactive double bonds of the metathesis starting materials and wherein the ethylene or propylene generated in the course of the reaction is removed from the reaction mixture.

2. Method according to claim 1, wherein the metathesis catalyst is selected from the group consisting of WCl_{6/}SnBu₄, WOCl₄/EtAlCl₂, MoO₃/SiO₂, Re₂O₇/Al₂O₃, and catalysts of general formulas 1, 2, and 3.

3. Method according to claim 2, wherein the metathesis catalyst is a ruthenium catalyst of general formula 3.

4. Method according to claim 3, wherein the metathesis catalyst is selected from formulas A, B, C and D.

5. Method according to claims 1 to 4, wherein ethylene or propylene are removed from the reaction mixture by volatilization.

6. Method according to claim 5, wherein the reaction mixture is sparged with inert gas to remove ethylene or propylene.

7. Method according to claim 6, wherein the reaction mixture is continuously sparged with inert gas to remove ethylene or propylene.

8. Method according to claims 1 to 7, wherein the metathesis starting materials are olefinic compounds having at least one reactive double bond in the form of an α-olefin and/or β-olefin, with the proviso that not more than one β-olefin is present in the same molecule.

9. Method according to claims 1 to 7, wherein the metathesis starting material is an olefinic compound having two reactive double bonds in the form of two α-olefins or one α-olefin and one β-olefin.

10. Method according to claim 1, wherein the metathesis starting materials are olefinic compounds having at least one reactive double bond in the form of an α-olefin and/or β-olefin, with the proviso that not more than one β-olefin is present in the same molecule, and the concentration of the starting materials is in a range of from 0.2 mM to 400 mM, wherein further ethylene or propylene is removed by continuously sparging the reaction mixture with inert gas, and wherein the reaction temperature is in a range of from 20°C to 150°C

11. Method according to claim 10, wherein the metathesis starting material is an olefinic compound having two reactive double bonds in the form of two α-olefins or one α-olefin and one β-olefin.

12. Method according to claim 10 or 11, wherein the metathesis catalyst is selected from the group consisting of WCl₆/SnBu₄, WOCl₄/EtAlCl₂, MoO₃/SiO₂, Re₂O₇/Al₂O₃, and catalysts of general formulas 1, 2, and 3.

13. Method according to claim 12, wherein the metathesis catalyst is a ruthenium catalyst of general formula 3.

14. Method according to claim 13, wherein the metathesis catalyst is selected from formulas A, B, C and D.

15. Method according to claims 1 to 14, wherein the metathesis reaction is a ring-closing metathesis reaction or cross-metathesis reaction.
